# EUROPEAN PATENT APPLICATION

(11) **EP 2 187 211 A1**
(43) Date of publication of application: **19.05.2010**
(21) Application number: 08169345.9
(22) Date of filing: 18.11.2008
(51) Int. Cl.: G01N 33/50

(54) **Diagnosis of diseases by measuring chemokinesis**

(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The present invention relates to the field of diagnosing diseases using cell-based assays. In particular the present invention pertains to the field of diagnosing coronary artery diseases by measuring random chemokinesis of cells.

## Description

### FIELD OF THE INVENTION

The present invention relates to the field of diagnosing diseases using cell-based assays. In particular the present invention pertains to the field of diagnosing coronary artery diseases by measuring random chemokinesis of cells.

### BACKGROUND OF THE INVENTION

It is well known that inflammation responses, specifically immune cells that produce various chemokines and growth factors, are involved in the development of a variety of diseases including coronary artery disease, diabetes, and various types of cancer such as e.g. breast cancer.

As a consequence, attempts have been made to elucidate the precise function of immune cells but also other disease-related factors during disease development.

Concurrently, with the increasing knowledge of the role of e.g. inflammatory cells and their produced factors during development of coronary artery diseases or different types of cancers, attempts have been made to use these factors as a diagnostic marker, either for ongoing disease development or the future-likely occurrence of the disease in question. One approach typically undertaken in this respect is to determine whether cellular expression of a disease-specific factor such as a growth factor is up-regulated or down-regulated compared to a control individual that is known not to suffer from the disease in question.

While these approaches allowed for significant improvements in the diagnosis and therapy of a multitude of diseases, there is a continuing need for diagnostic methods/test systems that allow a physician to decide on the disease state of a patient.

### OBJECT AND SUMMARY OF THE INVENTION

It is one objective of the present invention to provide novel approaches for diagnosing different types of diseases.

It is a further objective of the present invention to provide methods that allow obtaining data that can be used in diagnosing a variety of diseases.

These objectives as well as others which will become apparent from the ensuing description are attained by the subject-matter of the independent claims. Some of the preferred embodiments of the invention are defined by the dependent claims.

The present invention in one embodiment relates to a method of diagnosing and/or staging a disease comprising at least the following steps:
a. Providing at least one carrier structure wherein said carrier structure comprises a surface allowing for adherence and random migration of cells;
b. Providing cells derived from a sample of a human or animal being suffering from the disease in question;
c. Pre-Incubating said cells with a molecule that is capable of inducing a cellular activity in said cell;
d. Positioning said pre-incubated cells on said carrier structure;
e. Measuring the extent of chemokinesis undergone by cells deposited on said surface;
f. Providing control cells derived from a control sample of a human or animal being not suffering from the disease in question;
g. Pre-Incubating said control cells with a molecule that is capable of inducing a cellular activity in said cell;
h. Positioning said pre-incubated control cells on said carrier structure;
i. Measuring the extent of chemokinesis undergone by control cells deposited on said surface;
j. Comparing the results obtained in steps e. and i., and
k. Deciding on the occurrence and/or state of the disease.

Deciding on the occurrence and/or grade of the disease in step k. may be undertaken by different approaches. Thus, one may initially perform steps a. to k. for a patient in order to establish whether the patient suffers as such from the disease. Then, steps a. to e. may be repeated for the same patient at different times to establish a pattern for the extent of chemokinesis. If one observes a change in the extent of chemokinesis over time this may be an indication of ongoing disease development. Similarly, the method may be used to monitor response to therapy by determining the chemokinesis of cells derived from patients during different time points of medication.

In another embodiment the present invention relates to a method of data acquisition comprising at least the steps of:
a. Providing at least one carrier structure wherein said carrier structure comprises a surface allowing for adherence and random migration of cells;
b. Providing cells derived from a sample of a human or animal being suffering from the disease in question;
c. Pre-Incubating said cells with a molecule that is capable of inducing a cellular activity in said cell;
d. Positioning said pre-incubated cells on said carrier structure;
e. Measuring the extent of chemokinesis undergone by cells deposited on said surface;
f. Providing control cells derived from a control sample of a human or animal being not suffering from the disease in question;
g. Pre-Incubating said control cells with a molecule that is capable of inducing a cellular activity in said cell;
h. Positioning said pre-incubated control cells on said carrier structure;
i. Measuring the extent of chemokinesis undergone by control cells deposited on said surface; and
j. Comparing the results obtained in steps e. and i.

One may use the method of data acquisition again to either monitor a subject over a period of time by repeating steps a. to e. for the same patient and thus to stage the disease and/or monitor response to therapy and/or to compare the data with those obtained for a control only.

Typically, the carrier structure may be a flat carrier structure. In a preferred embodiment the carrier structure may be a microscopic slide made from materials such as glass.

The substrates in accordance with the invention are typically incubated with polymers that allow adherence and random migration of cells thereon. Such molecules may be of natural origin and include collagen, laminin, fibronectins, poly lysine, matrigel, lipid bilayers or may be purely synthetic polymers including polylactide, polylactide-co-glycolide or polycaprolacton, polystyrene, polyethylene, polyethylene glycol or may be combinations of polymers conjugated or adsorbed to various natural molecules.

In a typical embodiment, the disease to be diagnosed is selected from the group comprising coronary artery diseases, cancer, diabetes, autoimmune and inflammatory diseases/disorders. A particular focus of the present invention lies in the diagnosis of diseases in which angiogenesis is a central physiological process such as coronary artery diseases, diabetes, different types of cancer, inflammatory and autoimmune diseases such as rheumatoid arthritis constituting typical examples. Some of the preferred embodiments relate to the diagnosis of coronary artery diseases including diseases such as atherosclerosis.

In typical embodiments, cells may be selected from the group comprising embryonic, fetal or adults stem cells, progenitor cells, blood cells, fibroblasts, tumour cells and neuronal cells. The group of blood cells comprising monocytes, T and B lymphocytes, macrophages, eosinophils, basophiles and neutrophils can be preferred. A particular focus is on monocytes.

As pointed out in the claims such cells are pre-incubated with the molecule that is capable of inducing a cellular activity inside cells. Typically this refers to incubation with molecules being selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycans.

A particular focus is on molecules, which are capable of modifying signal transduction cascades in a cell such as growth factors, receptor ligands, hormones, cytokines, chemokines and cell adhesion molecules and integrins. A particularly preferred embodiment may relate to molecules, which are growth factors such as vascular endothelial growth factor (VEGF) or vascular cell adhesion molecule 1 (VCAM-1).

The methods of diagnosis, staging diseases and monitoring response to therapy and the methods of data acquisition in accordance with the invention rely on measuring random movement of cells, i.e. so-called chemokinesis. The methods in accordance with the invention thus do not look at the directed migration of cells towards known chemotactic stimulus over a distance in one direction (i.e. the direction of increasing or decreasing concentration of the stimulus), but rather observe the random migratory behaviour of cells after they have been contacted with the afore-mentioned molecules, which may, however, include pre-incubating cells with molecules that are know to induce a chemotactic response.

The extent of chemokinesis may typically be determined by measuring the ratio of randomly moving cells per total cells deposited on a given observation area on the substrate over a specified period of time and/or by determining the degree of displacement of cells from their individual cells origin on a given observation area over a specified period of time.

Some of the particularly preferred embodiments relate to methods of diagnosing, staging diseases and monitoring response to therapy and methods of data acquisition as outlined above in which the molecule, which induces a cellular activity is VEGF and/or VCAM-1, in which the cells are monocytes and in which the disease to be diagnosed is arteriosclerosis or atherosclerosis.

Other embodiments of the present invention will become apparent from the detailed description hereinafter.

### BRIEF DESCRIPTION OF THE DRAWINGS

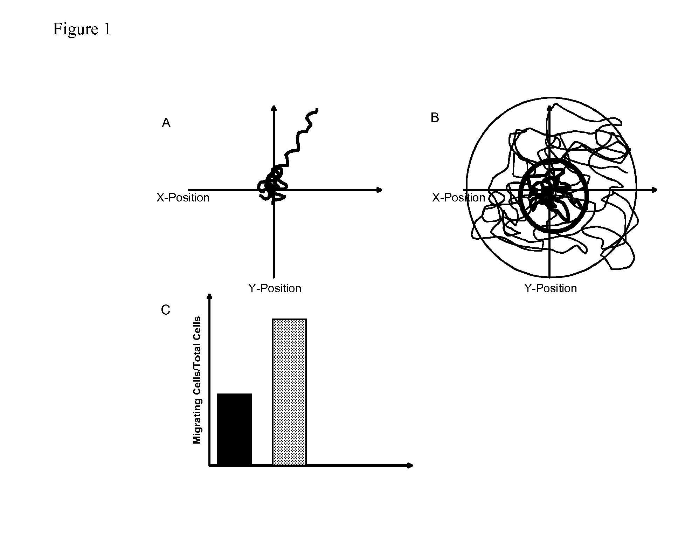

Figure 1 depicts the general concept of the present invention. Fig. 1A shows a comparison of a single cell tracking result for randomly migrating cell (thick bold line) and a cell that exhibits directed motion (chemotaxis, grey line). Fig. 1B shows the hypothetical result of migrating monocytes from a healthy patient with the VEGF pretreatment (grey line) and of a patient suffering from atherosclerosis, which have been pre-treated with VEGF (bold black line and build circle). When comparing a healthy individual to a sick individual one can notice a degree in the overall displacement of the monocytes in the tracking experiment reflective of a decreased random migration potential. Fig. 1C depicts the statistical analysis thereof.

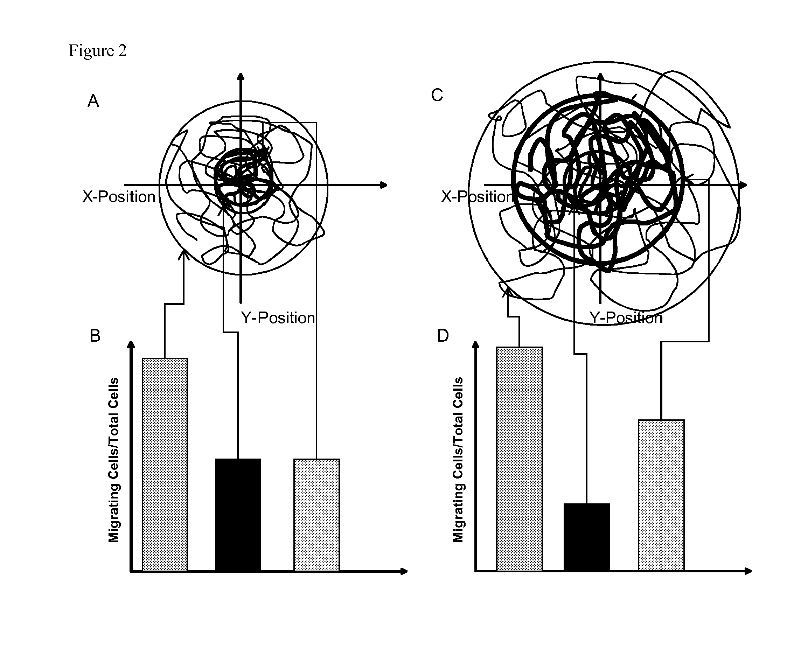

Figure 2 shows another comparison. Fig. 2A depicts the response to VEGF of cells of a healthy (thin grey line and thin outer circle, left bar of Fig. 2B) and two sick individuals suffering from cancer and coronary artery disease (CAD) (bold black line and thick grey line and respective circles, see also middle and right bar of Fig. 2B) are compared. Fig. 2C indicates the response of cells of the same patients, which have been pre-treated with VCAM-1. Again, the left bar of Figure 2D indicates the cells of a healthy human being while the middle and the right bar shows the cells of patients suffering from cancer (middle bar) or CAD (right bar).

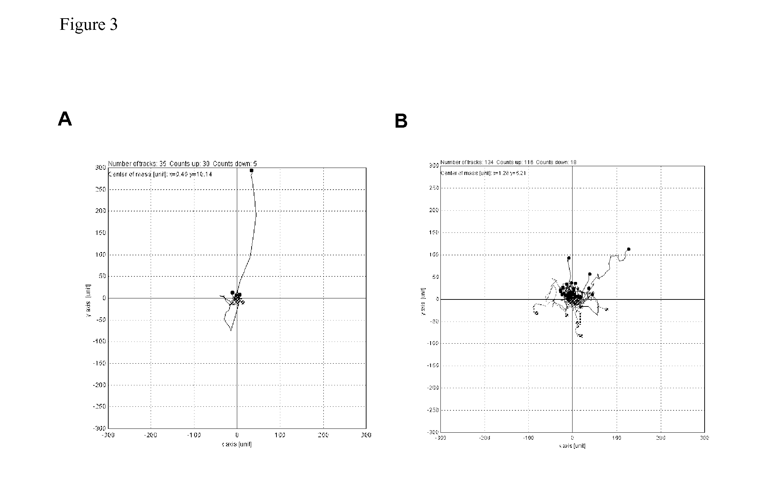

Figure 3 shows the effect of VEGF-treatment on random monocycte migration. Fig. 3A shows single cell tracking plots of 35 individual monocyte cells on fibronectin-coated surface without any pretreatment. Fig. 3B shows single cell tracking plots of 134 individual monocyte cells that were pretreated with 10 ng/mL of VEGF for 45 min prior to tracking on a fibronectin-coated surface.

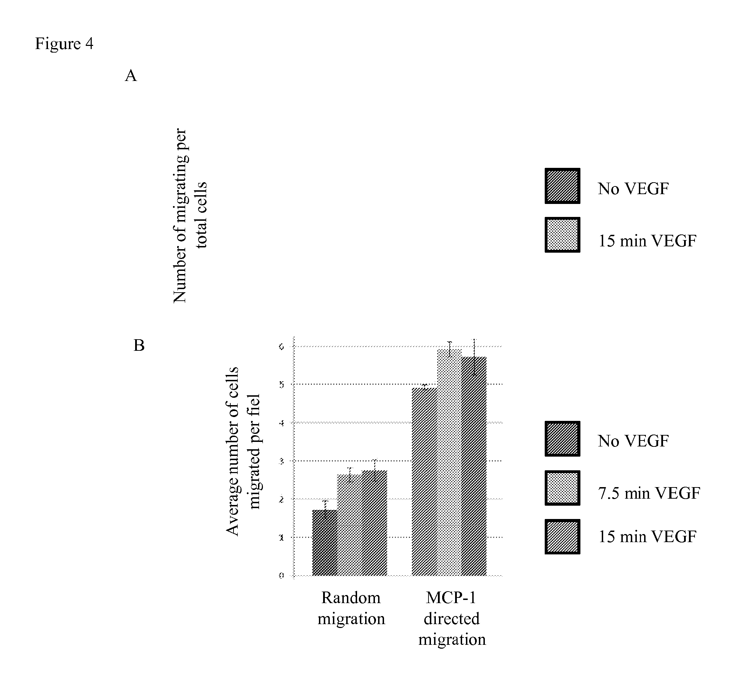

Figure 4 Fig. 4A depicts the ratio of net migrating monocytes per total monocyte cells for VEGF-untreated (left bar) and VEGF-treatment for 15 minutes with 10 ng/mL VEGF (right bar). Fig. 4B depicts modified Boyden chamber assay for monocyte chemotaxis directed against either no chemostimulus (random migration) or against 25 ng/mL MCP-1. Left bars indicate non-treated monocytes, whereas the middle and right bars indicate 10 ng/mL VEGF pretreatments for 7.5 and 15 min, respectively.

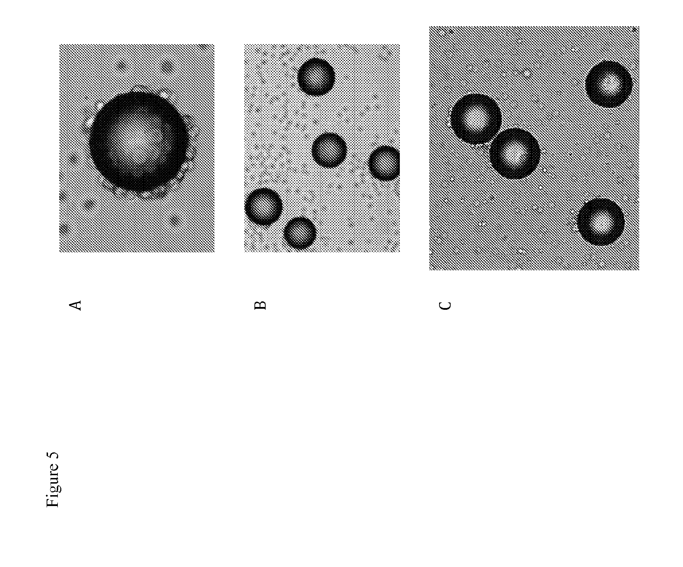

Figure 5 depicts adherence of cells to VEGF-functionalized microcarrier beads. Fig. 5A depicts the association of U937 monoblast cells to microcarrier beads, which have been functionalized with 1 ng/mL VCAM-1. Fig. 5B depicts control microcarrier beads without VCAM-1. Fig. 5C depicts binding of mononuclear cells isolated from whole human blood to microcarrier beads which have been functionalized with 1 ng/mL VCAM-1.

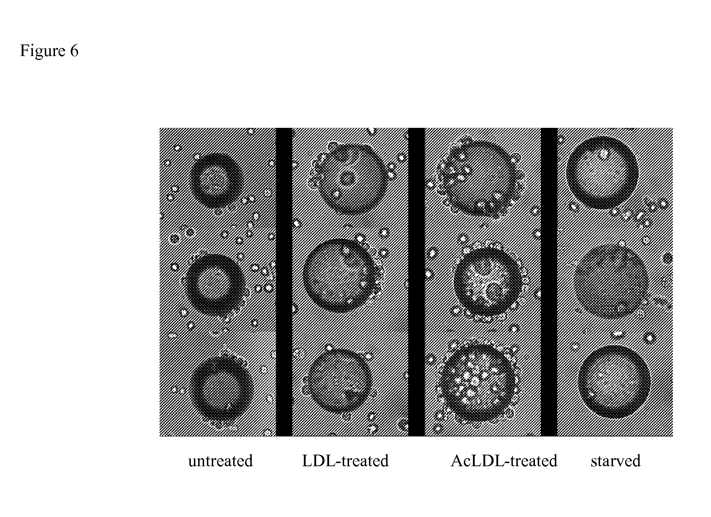

Figure 6 depicts binding of monocytes derived from the cell U973 to microcarrier beads loaded with VCAM-1. The left panel shows binding of normally grown monocytes. The second panel shows binding of monocytes cultivated in the presence of LDL. The third panel shows binding of monocytes cultivated in the presence of acetylated LDL. The right column shows binding of starved monocytes. The left axis indicates the average number of cells per bead.

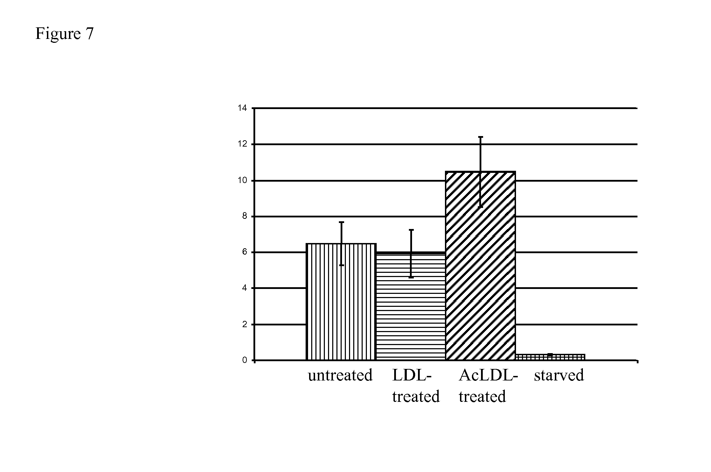

Figure 7 depicts the quantitative evaluation of the experiment highlighted in Figure 5. The data for each panel are from 3 independent experiments wherein 20 beads were counted for each experiment.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings as described are only schematic and nonlimiting. In the drawings, the size of some of the elements may be exaggerated and not drawn on scale for illustrative purposes.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. The terms "about" or "approximately" in the context of the present invention denotes an interval of accuracy that the person skilled in the art will understand to still ensure the technical effect of the feature in question. The term typically indicates deviation from the indicated numerical value of ±10%, and preferably of ±5%.

Further definitions of terms will be given in the following in the context of which the terms are used.

The present invention lays in part in the finding of the inventors that one can use random migration of cells on surfaces that allow attachment and migration of cells for diagnostic purposes if the cells analyzed have been challenged before with a molecule that is known to induce a cellular activity being typically observed in a disease.

Without wanting to be bound to a specific theory it is assumed that cells derived from a healthy individual or cells derived from a sick individual will differ in their response to stimuli that typically can be used to induce cellular activities. Such stimuli include e.g. molecules acting on signal transduction cascades with typical examples for this molecules being growth factors, cell adhesion molecules etc. The different effect of the same molecules on cells derived from healthy versus sick individuals can then be used in form of a readout that does not focus on the specific cellular activity being induced but rather on random migration, i.e. chemokinesis of the cells.

The present invention in one embodiment thus relates to a method of diagnosing and/or staging a disease comprising at least the following steps:
a. Providing at least one carrier structure wherein said carrier structure comprises a surface allowing for adherence and random migration of cells;
b. Providing cells derived from a sample of a human or animal being suffering from the disease in question;
c. Pre-Incubating said cells with a molecule that is capable of inducing a cellular activity in said cell;
d. Positioning said pre-incubated cells on said carrier structure;
e. Measuring the extent of chemokinesis undergone by cells deposited on said surface;
f. Providing control cells derived from a control sample of a human or animal being not suffering from the disease in question;
g. Pre-Incubating said control cells with a molecule that is capable of inducing a cellular activity in said cell;
h. Positioning said pre-incubated control cells on said carrier structure;
i. Measuring the extent of chemokinesis undergone by control cells deposited on said surface;
j. Comparing the results obtained in steps e. and i., and
k. Deciding on the occurrence and/or state of the disease.

As mentioned above, one may repeat a. to e. for a patient at different times to establish a pattern for the chemokinesis of this respective patient. If one observes a change in the extent of chemokinesis over time this may be an indication of ongoing disease development and may allow staging of the specific state of the disease.

The above-mentioned methods may also be used to monitor therapy. As far as monitoring progress of therapy is concerned one may take cells from a patient who suffers from the disease in question and who is taking corresponding medication. The comparison with cells from a healthy individual and/or the comparison with cells taken from the same sick individual taken at different points in time may allow to monitor responsiveness to therapy.

In another embodiment the invention relates to a method of data acquisition comprising at least the steps of:
a. Providing at least one carrier structure wherein said carrier structure comprises a surface allowing for adherence and random migration of cells;
b. Providing cells derived from a sample of a human or animal being suffering from the disease in question;
c. Pre-Incubating said cells with a molecule that is capable of inducing a cellular activity in said cell;
d. Positioning said pre-incubated cells on said carrier structure;
e. Measuring the extent of chemokinesis undergone by cells deposited on said surface;
f. Providing control cells derived from a control sample of a human or animal being not suffering from the disease in question;
g. Pre-Incubating said control cells with a molecule that is capable of inducing a a cellular activity in said cell;
h. Positioning said pre-incubated control cells on said carrier structure;
i. Measuring the extent of chemokinesis undergone by control cells deposited on said surface; and
j. Comparing the results obtained in steps e. and i.

In one embodiment, one may repeat steps a. to e. to establish how the data observed change over time and thus.

As far of the method of data acquisition is applied in the context of monitoring progress of therapy, one may take a sample with cells from a patient who suffers from the disease in question and who is taking corresponding medication. The comparison with the cells from a healthy individual and/or from cells from the same sick individual taken at different points in time may allow for the monitoring of responsiveness to therapy.

In one embodiment all afore-mentioned steps of the methods mentioned hereinafter are performed outside the human or animal body.

The methods for diagnosing and/or staging a disease and/or monitoring responsiveness to therapy as well as the methods for data acquisition will be discussed in conjunction hereinafter with respect to specific embodiments.

Substrates used for the invention can be of any geometric shape. The substrate may e.g. be planar or spherical (e.g. be a bead). It may also be present e.g. in the form of particles, strands, sheets, tubing, spheres, containers, capillaries, plates, microscopy slides, beads, membranes, filters etc.

In a preferred embodiment the substrate is planar and transparent.

In a further preferred embodiment suitable materials for the substrate include e.g. glass, plastic, nylon, silica, metal or polymers.

In a particularly preferred embodiment planar and transparent substrates made from polymers and/or glass are used. Suitable polymers for the substrate are e.g. cyclic olefin polymer (COP) or cyclic olefin copolymer (COC). A typical example for such a particularly preferred embodiment is a microscope slide.

The carrier structures in accordance with the invention may also be microcarrier structures.

The microcarrier structures may have any form that is suitable for the purposes of the present invention. They may thus have a rectangular shape, a triangular shape etc. Beads with round or substantially round form can be preferred according to the present invention. Thus, the carrier structures of the present invention in a preferred embodiment will typically have a round, elliptical or spheroid form.

While the size of the carrier structures is not critical, it can be preferred to use carrier structures that are in the micron size range. These carrier structures will be designated as microcarrier structures. Typically, microcarrier structures will have a diameter in the range of approximately 20 µm to about 1000 µm. Diameters in the range of approximately 75 µm to approximately 300 µm may be preferred, as may be diameters in the range of approximately 100 µm to about 200 µm. However, diameters in the range of 120 µm to about 1800 µm or about 150 µm may also be preferred. If such microcarrier structures take the aforementioned preferred round, elliptical or spheroid form they may be designated as microcarrier beads.

There are no specific requirements to the components or the interior structure of the microcarrier structures or beads except that the structures or beads must be capable of allowing a probe molecule to associate therewith and must be capable of allowing cells to associate with the probe molecules.

With respect to the aforementioned requirements, e.g. microcarrier beads may be produced from materials such as polystyrene, polydivnylbenzene and polymethylmethacrylate and biodegradable polymers such as poly-lactides, polyclycolides, polycaprolacton and copolymers thereof.

Such beads are in principle commercially available from e.g. SoloHill Engineering, Inc (Ann Arbor, Michigan, USA).

Other materials suitable for microcarrier beads include heparinised beads. The major polymers used in such bead manufacture are polystyrene and acrylic, and are commercially available.

The beads may be further modified, e.g. coated in order to ensure adherence of probe molecules and/or to allow for association of a certain type of cell (see below).

The person skilled in the art is familiar with the production of microcarrier beads. Thus, one may obtain these beads from aforementioned commercial sources or produce them according to the protocols being described in e.g. Gu et al (J. Control Release (2004), 96.3:463-472), Li et al (Acta Pharmacol. Sin. (2006) 27.6:754-759), Norrgren et al (Dev. Biol. Stand. (1983), 55:43-51) or Tatard et al (Biomaterials (2005), 26.17:3727-3737). Thus, the bead technology to create both e.g. microcarrier beads as well as probe covered-beads is known to the skilled person.

Microcarrier beads can e.g. be synthesized e.g. by controlled emulsification techniques such as submerged ink-jet printing.

If a microcarrier bead is to be produced from a polymer such as polystyrene, a polymer solution in a solvent such as dichloromethane can be ink-jetted into an aqueous phase containing a stabilizer for the monodisperse emulsion that is formed. To this end, one can use e.g. polyvinyl alcohol but also proteins could be used to achieve efficient stabilization. Subsequently polymer beads with a narrow size distribution are formed upon removal of the solvent.

The carrier structures in accordance with the present invention comprise a surface allowing for adherence and random migration of cells.

Such surfaces will typically be made from synthetic or natural polymers that allow attachment of cells and random migration thereon.

As far as synthetic polymers are used for these surfaces they may e.g. be selected from the group of polymers comprising polylactide, polylactide-co-glycolide or polycaprolacton, polystyrene, polyethylene and polyethylene glycol.

If the polymers used for the surfaces on the substrates are natural polymers they may be selected e.g. from poly-lactic acid, collagens, fibronectins, fibrins, laminins, elastins and/or nidogens. The use of fibronectins can be preferred.

Typically, such polymer surfaces will be deposited on the substrate by methods commonly known to the person skilled in the art. In case of natural polymers and particularly collagen, fibronectins, laminin, fibrins etc., the person skilled in the art will obtain these polymers from commercial sources and will then apply these natural polymers on the surfaces of the substrates in form of a solution and evaporate the solution.

The thickness of such polymer layers can be on the order of nanometers to micrometers Coatings on the nanometer (5-100 nm) scale can be preferred due to cost, as well as ability to penetrate with light (i.e. optical imaging and tracking).

Modification of a substrate with polymers such as those mentioned above may be achieved by covalent or non-covalent interaction between this substrate and the polymer. If polymers such as collagens, laminins, fibronectins etc. are used and should be covalently coupled to the substrates, substrates may be used that provide functional groups such as carboxyl groups, amine groups, hydroxyl groups, sulfid groups etc. These groups may then be cross-linked either directly to the polymers or one may use a linker that may be homo- or hetero-bi-functional.

Thus, substrates may be activated for providing a chemical linkage to the polymers such as fibronectins by e.g. coating the beads with a polymer having e.g. acyl fluoride functionalities. Other covalent attachment chemistries are also applicable but not limited to anhydrides, epoxides, aldehydes, hydrazides, acyl azides, aryl azides, diazo compounds, benzophenones, carbodiimides, imidoesters, isothiocyanates, NHS esters, CNBr, maleimides, tosylates, tresyl chloride, maleic acid anhydrides and carbonyldiimidazole.

Cells, which are to be examined by the methods in accordance with the present invention may be taken from different types of samples. Samples include e.g. tissue, blood, serum, feces, urine, hair, sputum etc.

In the context of the present invention the term "cell" typically refers to cell types that are known to be involved in the development of certain diseases such as coronary artery diseases, cancer, inflammatory diseases, diabetes etc. Preferably, one may use cells, which will typically be activated by specific cellular factors during disease development to migrate and/or adhere to certain cellular factors or structures. Thus, the migratory behaviour of cells may differ for e.g. blood samples derived from sick vs. healthy subjects. It is for example known that e.g. vascular endothelial growth factor is involved in angiogenesis. More specifically, endothelial cells in the arterial wall secrete VEGF, which then acts as a signal diffusing away from the site of excretion and establishing a gradient that diminishes as one moves away from the endothelial cells. This VEGF gradient acts on other endothelial cells as well as on cells being present in the blood such as monocyte cells. Typically, the monocytes will respond to VEGF gradients by moving towards increasing concentration of VEGF signals. After the monocytes have adhered to the endothelial cells producing the VEGF, they can start a plethora of signal mechanisms that ultimately result various responses including angiogenesis, tissue remodelling, inflammation, cell differentiation and phagocytosis.

It has further been observed that recruiting of blood cells including monocytes and lymphocytes contribute to the development of coronary artery diseases as well as of the metastasis and angiogenesis of cancer tumors.

Cells in accordance with the invention may thus be selected from the group comprising embryonic, fetal or adult stem cells, progenitor cells, blood cells, fibroblasts, tumour cells and urinal cells. Cells in accordance with the invention may not include human embryonic stem cells.

For the purposes of the present invention, the use of blood cells is preferred. Typically these blood cells comprise B and T lymphocytes, monocytes, macrophages, granulocytes, neutrophils, basophils and eosinophils.

Blood cells that have an immunological function such as monocytes, T-lymphocytes and neutrophils can be preferred. In one embodiment, cells relate to monocytes which have an immunological function and which will not further differentiate into endothelial cells so that these cells may not be considered as endothelial progenitor cells. However, monocytes may still differentiate into e.g. macrophages. Preferred are circulating, mature monocytes, although blood cell precursors, such as monoblasts are also usable

As has been mentioned above, the methods in the present invention may be used in the context of diagnosing and/or staging a disease. As also pointed out above, the diseases to be diagnosed may be diseases for which an implication of a specific cell type is known or suspected and which are **characterised in that** a specific cell type is recruited during disease development by a specific factor that may be used in the context of the present invention as the "inducing" or "activating" molecule.

Typically, diseases in the context of the present invention include e.g. coronary artery diseases such as atherosclerosis and arteriolosclerosis, diabetes, as well as pulmonary artery diseases, inflammatory diseases such as rheumatoid arthritis, and different types of cancers such as colorectal, lung, prostate and breast cancer. Other cancers may include leukemia, lymphoma, head and neck cancer, non-small cell lung cancer, ovarian cancer, urinary bladder cancer, kidney cancer, cervical cancer, or any solid state tumors etc.

Particularly for coronary artery diseases such as atherosclerosis and certain types of cancer, it is known that blood cells such as monocytes and macrophages play a major contributory role during disease development. For example in atherosclerosis, monocytes are recruited by cellular factors such as VEGF and VCAM-1 and then invade into the epithelium. Similarly, in certain types of cancer, tumour formation is characterised by an aberrant increase of angiogenic activity that is necessary in order to ensure that the tumour receives sufficient blood supply.

According to the methods of the present invention cells derived from samples will be pre-incubated with molecules being capable of "inducing" or "activating" a cellular activity in these cells.

It has already been mentioned above, that cells in accordance with the invention will typically be activated by specific cellular factors during disease development to display different characteristics depending on whether the cells are derived from a healthy or sick individual.

The term "molecule that is capable of inducing/activating a cellular activity" according to the present invention is to be understood in the broadest sense meaning that it refers to any type of molecule that is capable of inducing a response, e.g. an up- and/or down-regulation of signal transduction cascades, an up- and/or down-regulation of adhesion of cells to other cellular structures, an up- and/or down-regulation of metabolic turnover etc. Typically, one will use molecules which are know to act on signal transduction pathways such as growth factors, receptor ligands etc.

Molecules that are capable of inducing a cellular activity in cells are thus typically selected from the group comprising small molecules, peptides, glycopeptides, proteins, glycoproteins, receptors, receptor ligands, antibodies, growth factors, hormones, cytokines, chemokines, cell adhesion molecules, selectins, integrins, vitamins, nucleic acids and derivatives thereof, lipids and glycanes.

Growth factors, cell adhesion molecules, cytokines, chemokines and integrins may be preferred as probe molecules. Typical growth factors include e.g. human growth factor, human nerve growth factor, platelet derived growth factor, TGFβ, EGF, VEGF, TNF-a,TNF-b, FGFs, TGF-a, Epo, IGF-I, IGF-II, IL-1, IL-2, IL-6, IL-8, INF-g and CSFs as well as integrins. Other growth factors include CCL 1-28, CXCL 1-16, XCL1, XC:2, CX3CL1, IL 1-22, INF α, β and γ, M-CSF, G-CSF, GM-CSF, MIF, any recognized CD marker of hematopoetic cells (CD 1-339), PDGF, NGF, TPO, GDF-8, GDF-9, bFGF, HGF, FGF, DII4 and extracellular matrix components such as collagen, fibronectin and laminin. Likewise, cell adhesions molecules include e.g. VLA-1 to VLA-6, LPAM-1, LFA-1, CR3, CR4, leukointegrin, collagen, laminin, fibronectin, VCAM-1, MAdCAM-1, E-cadherin, P-selectin, L-selectin, E-selectin, CD62, ICAM-1, ICAM-2, ICAM-3, C3b and C4b.

Particularly preferred according to the present invention are growth factors and cell adhesion molecules with VEGF and VCAM-1 being preferred examples.

Pre-incubation of cells and the above described inducing/activating molecules will be performed by contacting the cells with the molecules. To this end, one typically will contact the cells and the molecules in appropriate buffer systems and incubate the mixture over a sufficient period of time to allow an interaction of the molecules with the cells.

The contacting or incubation step may typically take from about 1 minute to about 720, from about at least 5 to about at least 600 minutes, from about at least 10 to about at least 300 minutes or from about at least 5, 10, 15, 20, 25 or 30 minutes to at least about 40, 50, 60, or 120 minutes.

Depending on the cell type to be analyzed it may be advisable to pre-purify the cells from the sample before contacting it with the molecules that are used to activate a cellular activity within the cell. The person skilled in the art knows how to prepare and obtain such samples and how to pre-purify cells.

For example one may draw blood from a patient and perform a buffy-coat centrifugation to isolate mononuclear cells resulting in a mixture of lymphocytes and monocytes. As to whether and to what extent pre-purification of the sample will be necessary depends then on type of cells to be analysed as well as on the type of disease to be diagnosed. Purified monocytes are easily available via commercial negative selection magnetic cell sorting kits (Miltenyi Biotec, BD Biosciences).

The concentration of molecules that are capable of inducing a cellular activity to be used on the respective cells will depend on the specific molecule in question.

For VEGF the concentration range will typically be in the range from about 0.01 ng/mL to about 1 µg/mL. The preferred levels will be in the range of about 0.5 to about 100 ng/mL and more preferably in the range of about 1 to about 20 ng/ml or about 1 to about 10 ng/ml. The above concentrations typically refer to pre-incubation of cells in the range of about 1x10⁴ to 1x10⁷ cells. Particularly preferred are VEGF concentrations of about 5 to 10 ng/mL if 0.5-1x10⁶ cells/mL are to be contacted. The incubation time in this case may typically be on the order of 5 to 60 minutes, with a preference for 10-15 min.

For VCAM-1 the typical concentrations will be in the range of 0.01 ng/mL to about 100 µg/mL, with a preference of about 1-10 µg /mL or 10-100 µg /mL.

As pointed out above, the methods in accordance with the invention require then determining the extent of chemokinesis undergone by the pre-incubated cells deposited on the substrates.

In the context of the present invention the term "chemokinesis" relates to the random migratory behaviour of a cell.

A lot of cell types are known to move towards or away from certain chemical stimuli depending on whether these chemical stimuli are considered to be chemoattractants or chemorepellants. This directed migration towards or away from a certain molecule over a long distance is typically be considered as chemotaxis.

The present invention thus to some degree relies on the finding that one cannot only use the directed migration of cells towards certain stimuli (i.e. chemotaxis), but actually trandom migration of cells (i.e. chemokinesis) for diagnostic purposes. The person skilled in the art is familiar how to differentiate chemotactic versus random migratory behaviour of cells.

Chemotactic behaviour is characterized directed displacement of a cell, typically over a distance of at least 2 fold the diameter of the cell (hence a minimum of 20 µm and above) in a specific direction, with respect to a chemically induced gradient of a signal (i.e. VEGF).

Chemokinesis is rather characterized in movement from the cells origin over similardistances as in chemotaxis, however the direction of the motion is random and not in response to any chemically induced gradients of stimulus (i.e. the surrounding medium is uniform).

Typically, a chemotactic behaviour will be characterized by displacement over at least 2-5 times at cell diameter (being typically in the range of 15 to 20 µm) into one direction, i.e. the direction to or away from the chemotactic stimulus. Instead, typical random migratory behaviour is characterized by a cell's displacement over 1 to 5 times of its cell diameter into all directions.

The person skilled in the art will understand that the distance migrated by cells in all directions will depend on the observation period. The extent of chemokinesis determined in accordance with the present invention must therefore be correlated with the time over which chemokinesis is recorded. This time will typically be in the range of at least about 1 to about 60 minutes, preferably at least about 1 to about 50 minutes, preferably at least 1 to about 40 minutes, more preferably at least 1 to at least about 30 minutes, at least about 1 to about 20 minutes and even more preferably at least about 1 to about 15 minutes or at least about 1 to about 10 minutes.

In case that there are doubts as to whether the migratory behaviour observed is indeed random or rather chemotactic, one may perform suitable control experiments by e.g. subjecting the cells to a known chemotactic stimulus. The response induced by such a chemotactic stimulus will show the skilled person whether the behaviour observed beforehand was indeed random or chemotactic.

In order to determine the extent of random migratory behaviour, i.e. chemokinesis the person skilled in the art may determine the ratio of randomly moving cells per total cells deposited on a given observation area for a given period of time.

Additionally and/or alternatively, the extent of chemokinesis may be measured determining the degree of displacement of cells from their individual origin over a given observation area and given period of time.

As will become apparent from the ensuing examples, cells derived from patients versus healthy individuals may be **characterized in that** the extent of chemokinesis significantly differs. For example, cells from a healthy individual may be characterized by a random migratory behaviour wherein the cell migrates over a distance corresponding up to 5 times the cells' diameter in all directions. In comparison a cell derived from a patient may undergo random migration in all directions, but only over a distance corresponding to 1-2 times the cells' diameter.

Thus, one may use the degree of displacement of a cell from its origin, i.e. the spot on the substrate where it was originally located as an indication of its migratory behaviour. Using common monitoring techniques such as video trapping or time lapse video microscopy, one can follow the movement of a cell and determine its average displacement distance in all directions for a given period of time.

The person skilled in the art will understand that for the methods in accordance with the invention it may be preferable to analyze at least a certain number of cells from healthy and sick individuals in order that the random migratory behaviour observed is indeed a reliable indicator.

Thus, the random migratory behaviour may preferably be characterized for at least about 25, at least about 50, at least about 100, at least about 150, at least about 200, at least about 250, at least about 300, at least about 350, at least about 400 or at least about 500 cells derived of healthy or sick individuals. Typically, the results obtained will be more reliable if more cells are observed. It can be expected that an analysis of at least 25-50 cells will give reliable examples and that counting up to 100 cells should be sufficient.

The random migratory behaviour obtained for these multiple number of cells will then be averaged and the average migration distance in all directions over a give period of time will be characteristic of the respective cell type. This parameter may be designated as "average displacement distance" in all directions for a given period of and may be visualized as a "shell distance" assuming that all cells were put initially on the spot. The "average displacement distance" or "shell distance" can be used to describe the extent of chemokinesis as measured by the methods in accordance with the invention.

With the "average displacement distance" (i.e. movement distance over time in all directions) at hand, cells may be differentiated. Thus, the "average displacement distance" may be used to differentiate between different cells types which have been pre-incubated with the same molecule and have been derived form the same type of individuals, this parameter may be used to differentiate between identical cell types from identical type of individuals being treated with different molecules or it may be used to differentiate between identical cell types that have been treated with identical molecules but with the cells being derived from either sick or healthy individuals, etc.

Instead of using the "average displacement distance" as a parameter indicative of the extent of chemokinesis, one may also simply determine the numbers of randomly or even all (i.e. including chemotactic) migrating cells per defined observation space and time versus the total number being deposited in that observation area. The ratio may be used for the purposes of the present invention to assess the extent of chemokinesis. A decision as to whether a cell will be considered to be chemokinetic or chemotactic will again be made based on the fact whether cells migrate over comparatively small distances in all directions or larger distances only in one direction.

In order to determine the extent of chemokinesis, one may thus rely on different parameters such as the "number of chemokinetic or all migrating cells vs. total cells" or the "average displacement distance". Both of these parameters describe either the amount of cells undergoing random versus directed migration or the extent by which cells undergo random versus directed migration.

The invention is described hereinafter in terms of experiments, which relate to some of the preferred embodiments of the present invention. These experiments are not to be construed as limiting the invention in any way.

### Hypothetical experiment 1

In the following hypothetical experiment, it is described how pre-incubation of monocytes with VEGF is undertaken and how measurement of chemokinesis is used to differentiate between cells derived from healthy or sick individuals.

The assay is performed by mixing an appropriate concentration of enriched mononuclear blood cells (usually on the order of 0.5 - 1x10⁶ cells/mL) with an appropriate VEGF concentration (typically around 5-10 ng/mL) for various times (usually on the order of 20-30 min). Thereafter, the cells are deposited onto the surface of a microscope slide. The microscope slide can contain e.g. fibronectin on its surface. As a control, monocytes that have not been treated with VEGF can be deposited on an adjacent slide or region of the same slide.

The cells are allowed to settle onto the slide for about 5 min and are then imaged (usually at a rate of one image every 30-60 seconds for 30-60 minutes).

Following these image captures, the images are compressed to make a short video clip of the migrating cells in which individual cells can be examined and tracked. Typically, at least 100 cells will be tracked. Figure 1A demonstrates a typical "tracking trajectory" of a single cell, in both a 'random motion'- (chemokinesis, black line) and a 'directed motion' (chemotaxis, grey line) type scenario. Figure 1B then demonstrates a typical experiment in which 2 cell populations are demonstrated for random walking, population 1 in grey is a healthy monocyte pre-treated with VEGF and demonstrates an increased migration potential. Population 2 in black signifies a monocyte from an unhealthy patient (say an obese, smoking individual with CAD) that was also pretreated with VEGF. Further, for control purposes monocytes from the healthy individual are analysed which have not been pre-treated with VEGF (not shown).

While population 2 monocytes do show more migration activity that non-VEGF activated monocytes (control population, not shown), the "average ckemokinesis distance" shell (black circle) is noticeably smaller than that of the healthy patient population 1 (grey circle). This shell (area of the circle) is indicative of the "average chemokinesis distance" of the cells from their deposited origin.

Furthermore, the overall number of migrating cells (defined by cells that have displaced at least one cell diameter, i.e. about 10-20 µm) per total cells analyzed also gives an indication of random migratory capacity of the cells (Figure 1 C).

Thus, non-VEGF treated monocytes (not shown) do not migrate significantly, since they are not primed to do so, however, the difference between a sick patient (grey) and a healthy individual (black) can in principle be elucidated from such an approach.

### Hypothetical experiment 2

In the following hypothetical experiment, it is described how pre-incubation of monocytes with VEGF is undertaken and how measurement of chemokinesis is used to differentiate between cells derived from healthy or sick patients suffering from different diseases.

As explained, the chemokinesis parameters can reveal patient specific information. Since it can be argued that e.g. monocyte migration induced by VEGF is impaired in many disease states, such an analysis may allow for discrimination of the monocyte-VEGF function between various disorders. That is, the levels of expression of VEGF and the ability of a patient's monocytes to respond to those levels are individually and disease state based. By testing a range of VEGF concentrations, or various substrates on the microscope slides, at a constant concentration of patient blood cells, trends may be elucidated that may not be evident using traditional migration assays. This may not only apply to VEGF and monocytes, but also to different cell types and activiation molecules. Further, by challenging cells not only with one agent (e.g. VEGF), but also with different agents (such as VCAM-1), different diseases may be diagnosed (see Figure 2).

In this example, the response to VEGF between a healthy (dark grey line) and sick (black and light grey lines) patient is evident using only VEGF treatment and a fibronectin-coated microscope slide, however, the differences between two sick patients may not be so clear (i.e. a Lung Cancer (black line) patient and a coronary artery disease (CAD) patient (light grey line) (see Figure 2A and B, middle and right bar respectively).

Since VCAM-1 is also upregulated during CAD development, at the site of the atherosclerotic plaque, the response of VCAM-1 pre-treated monocytes of the patient with CAD on a fibronectin-coated microscope slide may be distinguished from a patient with Lung Cancer (see Figure 2C and D, middle and right bar respectively).

### Experiment 3

In the following experiment, it is described how pre-incubation of monocytes with VEGF is undertaken and how pre-treatment has an effect on chemokinesis.

Monocytes were isolated via negative selection methods from human blood. These monocytes were left either untreated in a balanced salt solution, or pretreated for 45 min with 10 ng/mL VEGF, on ice. Then these two monocyte samples were seeded onto a fibronectin coated microscope slide at approximately 5x10⁵ cells/mL and their random migration was observed for 45 min with time lapse microscopy (35 untreated monocytes and 134 VEGF pre-treated monocytes).

As one can see from the projection plots in Figure 3, monocytes that have been pretreated with VEGF are much more "active" (i.e. randomly migrate to a greater extent and/or a greater distance) than those untreated.

Furthermore, it has been established before that within 5 minutes of treatment, monocyte signal transduction cascades are induced by VEGF.

Hence, the above experiment was repeated with only 15 minutes of VEGF pre-treatment (versus non-treated monocytes).

In Figure 4, the data are presented as a ratio of migrating cells/total cells. Again one can clearly see that in the case of VEGF pre-treatment, more cells migrate as a whole, than versus non-treated.

Hence if an individual's monocytes do not respond well to VEGF (as is the case during disease development), then one would expect to see both less net cell migration distance (as in Figure 3), but also, less cells migrating in total (as in Figure 4A).

As a control, the effects of VEGF pre-treatment were compared in the context of the "gold standard" cell migration assay, the modified boyden chamber assay as described in (Waltenberger, J., et al., Circulation 102.2 (2000): 185-90). Again, either monocytes, which had been pre-treated for 7.5 min or 15 min with 10 ng/mL VEGF or untreated monocytes were compared. These different cells were then tested for migration towards the chemokine MCP-1.

While no distinguishable differences between 7.5 and 15min pretreatment of VEGF (to be expected since VEGF signaling is maximal within 5 min at 10 ng/mL) were observed, it was clear that both random migration of monocytes as well as MCP-1 directed migration of monocytes was stimulated by VEGF pre-treatment (Figure 4B) and that chemokinesis can be clearly distinguished from chemotaxis.

### Experiment 4

The following experiment shows that monocytes derived from healthy individuals or from CAD patients should differ in their responses to factors such VEGF and VCAM-1.

Microcarrier were commercially obtained from Solohill (www.solohill.com). Subsequently, VCAM-1 was coupled at a concentration of 1 ng/mL to the Solohill microcarrier beads using standard protein cross-linking. First the collagen coating on the beads was activated to form a reactive NHS-ester, which was then subsequently reacted with amine groups found on the N-terminal and lysine residues of the VCAM-1. As a control, a blank microcarrier bead without any coupling of VCAM-1 was used.

Subsequently 10 µL of 1 mg/mL microcarrier beads were reacted for 30 minutes with 2.5x10⁶ cells/mL of U937 cells.

Figure 5A and B show that only in case of VEGFvcam1? functionalization the U937 cells adhere to the microcarrier beads.

In a second experiment the same microcarrier beads were tested for binding to mononuclear cells, which had been isolated from whole blood by standard Ficoll plaque centrifugation to isolate mononuclear white blood cells directly from whole blood. Also in this case it was possible to detect a binding of the cells from the sample to the VEGF functionalized beads (see Figure 5C).

U937 cells that can be obtained from the ATCC were then grown in standard RPMI media (Invitrogen), following the provider's recommended culturing procedures.

Briefly, cells were grown under exponential growth phase conditions and passaged at sub-confluence 2-3 times per week into fresh medium.

In addition, cells were grown in the presence of low-density lipoprotein (LDL) or acetylated LDL (AcLDL) (Invitrogen) at a concentration of 20 µg/mL for 7 days. Acetylated LDL mimicks oxidized LDL the accumulation of which is indicative for patients suffering from atherosclerosis and is taken up by monocyte through the scavenger receptor. LDL, however, cannot be taken up monocytes as they lack a corresponding receptor.

Approximately 50-100 µl of these differently treated cells at a concentration of 1x10⁶ cells/mL were then incubated with 5 µL of 1 mg/mL microcarrier beads (Solohill) which had been cross-linked with human recombinant VCAM-1 (Biospacific).

VCAM-1 beads were prepared as follows: 500 mg of commercially available 90-150 µm collagen coated microcarrier beads were resuspended in 10 mL of 0.1 M 2-[morpholino]ethanesulfonic acid, 0.5M NaCl, pH 6.0. Bead suspension was activated by reaction with 4 mg of 1-ethyl-3-[3-dimethylaminopropyl]carbodiimide and 11 mg of N-Hydroxysulfosuccinimide with gentle inversion for 15 minutes at room temperature. Beads were washed by centrifugation for 2 minutes at 1000 rpm, supernatant removal, and subsequent resuspension in fresh phosphate buffered saline, pH 7. This washing procedure was repeated 5 times. 1 mL of the activated 50mg/mL bead suspension was then added directly to 200 µg of lyophilized, commercially available, recombinant, human VCAM-1 protein. This reaction was performed at room temperature, with gentle inversion for 3-5 hours. The reaction was quenched with via addition of 10 mM Hydroxylamine for 5 min, with gentle inversion at room temperature. Beads were washed 5 times as described above, via centrifugation, supernatant removal and resuspension in fresh PBS, pH 7. Microcarriers at an approximate concentration of 50 mg/mL are stored as 20 µL aliquots at -80 degrees C.

Next, 5 µL of VCAM-1 conjugated microcarriers is reacted with 50-100 µL of monocyte cells at a density of 1x10⁶ cells/mL for 10-15 minutes at room temperature. Following the reaction, 50 µL of the bead/cell suspension is pipetted onto a microscope slide and examined with a simple bright field microscope. Images are focused upon the equator of the microcarrier beads, such that only cells bound to this region are in focus. For each sample, an average 20-30 beads were examined and the cells upon those beads counted. Results can be expressed as a simple average cells/bead value.

It can be seen from Fig. 6 and 7 that cells that have been treated with AcLDL interact differently with VCAM-1 functionalized microcarrier beads.

Experiments 3 and 4 thus show that monocytes show different random migratory behavior depending on pre-treatment with VEGF and that monocytes derived from CAD patients and healthy individuals should differ as regards their response to VEGF and/or VCAM-1.

## Claims

1. Method of diagnosing and/or staging a disease comprising at least the following steps:
a. Providing at least one carrier structure wherein said carrier structure comprises a surface allowing for adherence and random migration of cells;
b. Providing cells derived from a sample of a human or animal being suffering from the disease in question;
c. Pre-Incubating said cells with a molecule that is capable of inducing a cellular activity in said cell;
d. Positioning said pre-incubated cells on said carrier structure;
e. Measuring the extent of chemokinesis undergone by cells deposited on said surface;
f. Providing control cells derived from a control sample of a human or animal being not suffering from the disease in question;
g. Pre-Incubating said control cells with a molecule that is capable of inducing a cellular activity in said cell;
h. Positioning said pre-incubated control cells on said carrier structure;
i. Measuring the extent of chemokinesis undergone by control cells deposited on said surface;
j. Comparing the results obtained in steps e. and i., and
k. Deciding on the occurrence and/or state of the disease.

2. Method of data acquisition comprising at least the steps of:
a. Providing at least one carrier structure wherein said carrier structure comprises a surface allowing for adherence and random migration of cells;
b. Providing cells derived from a sample of a human or animal being suffering from the disease in question;
c. Pre-Incubating said cells with a molecule that is capable of inducing a cellular activity in said cell;
d. Positioning said pre-incubated cells on said carrier structure;
e. Measuring the extent of chemokinesis undergone by cells deposited on said surface;
f. Providing control cells derived from a control sample of a human or animal being not suffering from the disease in question;
g. Pre-Incubating said control cells with a molecule that is capable of inducing a a cellular activity in said cell;
h. Positioning said pre-incubated control cells on said carrier structure;
i. Measuring the extent of chemokinesis undergone by control cells deposited on said surface; and
j. Comparing the results obtained in steps e. and i.

3. Method according to claim 1 or 2,
wherein said cells are selected from the group comprising embryonic, fetal or adults stem cells, progenitor cells, blood cells, fibroblasts, tumor cells and neuronal cells.

4. Method according to claim 3,
wherein said cells are selected from the group of blood cells comprising monocytes, T and B lymphocytes, macrophages, eosinphils, basophils and neutrophils.

5. Method according to claim 1 or 2,
wherein said molecule that is capable of inducing a cellular activiry is selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycanes.

6. Method according to claim 1 or 2,
wherein said disease is selected from the group comprising coronary artery diseases, cancer, diabetes, autoimmune and inflammatory diseases/disorders said.

7. Method according to claim 1 or 2,
wherein said molecule that is capable of inducing a cellular activity is a growth factor such as VEGF and/or VCAM-1, wherein said at least one cell is selected from monocytes and wherein the disease is a coronary artery disease such as atherosclerosis.

8. Method according to claim 1or 2,
wherein the extent of chemokinesis is measured by determining the ratio of randomly moving cells per total cells deposited on a given observation area.

9. Method according to claim 1 or 2,
wherein the extent of chemokinesis is measured by determining the degree of displacement of cells from the individual cells origin on a given observation area.
